(19) Europäisches Patentamt
European Patent Office
Office européen des brevets

(11) **EP 4 052 644 A1**

(12) **EUROPEAN PATENT APPLICATION**

(43) Date of publication:
**07.09.2022 Bulletin 2022/36**

(51) International Patent Classification (IPC):
***A61B 5/024*** (2006.01)   ***A61B 5/113*** (2006.01)
***A61B 5/00*** (2006.01)   ***A61B 5/11*** (2006.01)

(21) Application number: **21160464.0**

(22) Date of filing: **03.03.2021**

(52) Cooperative Patent Classification (CPC):
**A61B 5/6892; A61B 5/024; A61B 5/1102;
A61B 5/1135; A61B 5/7207; A61B 5/7257**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **BERA, Deep**
  **5656 AE Eindhoven (NL)**
• **SETH, Subhendu**
  **5656 AE Eindhoven (NL)**
• **LONG, Xi**
  **5656 AE Eindhoven (NL)**

(74) Representative: **Philips Intellectual Property &
Standards
High Tech Campus 52
5656 AG Eindhoven (NL)**

(54) **MEASURING A VITAL SIGN OF A SUBJECT**

(57)   An apparatus for measuring a first vital sign of a subject comprises a memory comprising instruction data representing a set of instructions and a processor configured to communicate with the memory and to execute the set of instructions. The set of instructions, when executed by the processor, cause the processor to: obtain signals from each of a plurality of sensors in a mat, wherein the plurality of sensors are suitable for use in measuring vital signs of a subject when the subject is lying on the mat; determine a power spectrum density of each signal in a first frequency range in which the first vital sign is detectable; and select a first sensor from the plurality of sensors with which to measure the first vital sign, based on the power spectrum densities.

Fig. 2

# Description

FIELD OF THE INVENTION

Embodiments herein relate to methods and apparatus for measuring vital signs of a subject. Some embodiments relate to measuring vital signs using a plurality of sensors in a mat, such as a sleep mat for a baby or neonate.

BACKGROUND OF THE INVENTION

Objective monitoring of vital signs for (term and preterm) babies/infants and immobile patients is important for healthcare professionals in order to be able to analyze and evaluate health conditions of the patient and in order to be able to take immediate action in the event that the vital sign indicates a potential problem. This is particularly important in intensive care situations.

The assessment of vital signs is often done by analyzing signals from several sensors attached to a patient's body. However, in many situations, it is not ideal to attach sensors directly to the patient's skin as this can cause severe discomfort; the skin of neonates and young infants can be particularly fragile, for example. Additionally, parents and caregivers find it difficult to handle patients connected to multiple wires. Moreover, if sensors become detached, reattachment can require expertise which can cause delays and interfere with patient monitoring.

For epileptic patients, video EEG assisted identification of seizure events is important for surgery planning. As such, unobtrusive seizure prediction and monitoring is also an important area of current research.

With respect to infant monitoring, infant monitors using various techniques have been developed for vital sign monitoring. For example, camera-based baby monitors have advantages of unobtrusiveness and ease-of-use. However, with video cameras, it can be difficult to obtain an accurate physiological data from the infant during sleep, which is crucial in separating active and quiet sleep.

Fibre optic sensors have also been suggested as an unobtrusive way of detecting an infant's respiration and movement without attaching any sensors to the infant's skin. This may be helpful to monitor for infant diseases and to prevent/reduce sudden infant death syndrome.

It is an object of embodiments herein to provide improved vital sign monitoring for subjects such as neonates, infants and patients with epilepsy.

SUMMARY OF THE INVENTION

As noted above, unobtrusive vital sign monitoring is highly desirable for patients such as neonates and infants as well as for epilepsy monitoring. Mats of sensors positioned underneath an infant, in an infant mattress or as part of an integrated matt in a sleep mattress are a promising solution because they are highly unobtrusive and yet can be sensitive enough to measure signals modulated by physical and/or physiological activities including body movement, respiration, and/or heartbeat. This signal can be measured, for example, using piezo-electric pressure sensory units, accelerometers, or proximity sensors. Such sleep mats have previously been used, for example to measure ballistocardiogram (BCG) signals for infant sleep monitoring. It is an object of embodiments herein to improve on such sleep mats and more generally, to improve on vital sign monitoring using pressure sensors.

According to a first aspect there is provided an apparatus for measuring a first vital sign of a subject. The apparatus comprises: a memory comprising instruction data representing a set of instructions, and a processor configured to communicate with the memory and to execute the set of instructions. The set of instructions, when executed by the processor, cause the processor to: obtain signals from each of a plurality of sensors in a mat, wherein the plurality of sensors are suitable for use in measuring vital signs of a subject when the subject is lying on the mat; determine a power spectrum density of each signal in a first frequency range in which the first vital sign is detectable; and select a first sensor from the plurality of sensors with which to measure the first vital sign, based on the power spectrum densities.

According to a second aspect there is a system for determining a vital sign of a subject. The system comprises a mat comprising a plurality of sensors suitable for use in measuring vital signs of a subject when the subject is lying on the mat, and an apparatus as in the first aspect.

According to a third aspect there is a method for measuring a first vital sign of a subject. The method comprises obtaining signals from each of a plurality of sensors in a mat, wherein the plurality of sensors are suitable for use in measuring vital signs of a subject when the subject is lying on the mat; determining a power spectrum density of each signal in a first frequency range in which the first vital sign is detectable; and selecting a first sensor from the plurality of sensors with which to measure the first vital sign, based on the power spectrum densities.

According to a fourth aspect there is a computer program product comprising a computer readable medium, the computer readable medium having computer readable code embodied therein, the computer readable code being configured such that, on execution by a suitable computer or processor, the computer or processor is caused to perform the method of the third aspect.

Embodiments herein improve the accuracy of vital sign measurements made using sensors arranged in a mat (such as a "sleep-mat") by selecting the optimal sensor with which to monitor the measurements, based on the power spectrum density of the signals received from each sensor. In this way, the strongest signal can be obtained (e.g. corresponding to the sensor in closest/optimal proximity to the subject) with which to obtain the most reliable measurements. There is thus provided unobtrusive automatic detection of vital signs with adaptive sensor selection for reliable vital sign estimation.

These and other aspects will be apparent from and elucidated with reference to the embodiment(s) described hereinafter.

BRIEF DESCRIPTION OF THE DRAWINGS

Example embodiments will now be described, by way of example only, with reference to the following drawings, in which:

Fig. 1 shows an apparatus according to some embodiments herein;
Fig. 2 shows a method according to some embodiments herein;
Fig. 3 shows a method according to some embodiments herein;
Fig. 4 shows output of a signal processing method according to some embodiments herein;
Fig. 5 shows a method according to some embodiments herein;
Fig. 6 shows a method according to some embodiments herein; and
Figs. 7a and 7b show the output of a signal processing method according to some embodiments herein.

DETAILED DESCRIPTION OF EMBODIMENTS

As described above, embodiments herein relate to improved processing of signals from a mat of sensors, such as a sleep mat, for use in measuring vital signs of a subject whilst the subject lies on the mat.

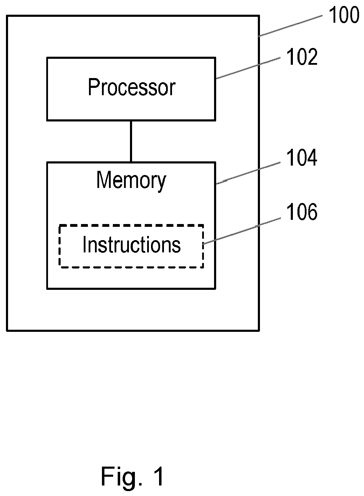

In more detail, turning to Fig. 1, in some embodiments there is an apparatus 100 for use in measuring a first vital sign of a subject, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the method 200 as described below.

Embodiments of the apparatus 100 may be for use in measuring vital signs of a subject. More specifically, the set of instructions, when executed by the processor, cause the processor to: obtain signals from each of a plurality of sensors in a mat, wherein the plurality of sensors are suitable for use in measuring vital signs of a subject when the subject is lying on the mat, determine a power spectrum density of each signal in a first frequency range in which the first vital sign is detectable, and select a first sensor from the plurality of sensors with which to measure the first vital sign, based on the power spectrum densities.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the signals from the plurality of sensors, the determined power spectrum densities and/or any other information or data received, calculated or determined by the processor 102 of the apparatus 100 or from any interfaces, memories or devices that are external to the apparatus 100. The processor 102 may be configured to control the memory 104 to store the signals from the plurality of sensors, and/or the determined power spectrum densities.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

In some embodiments, the apparatus 100 described above may form part of a larger system. For example, a system for determining a vital sign of a subject may comprise the apparatus 100. Such a system may further comprise a mat comprising a plurality of sensors suitable for use in measuring vital signs of a subject when the subject is lying on the mat.

In some embodiments, such a system is for monitoring vital signs of a baby or a neonate. The mat can be a sleep mat e.g. a mat associated with monitoring an infant or neonate whilst they are lying on the mat, e.g. resting/sleeping.

In other embodiments, the mat is for monitoring vital signs of a subject of any age, e.g. baby, child or adult. For example, the mat may be for monitoring a subject with epilepsy, for the purpose of seizure prediction.

More generally the mat may be comprised in any surface containing sensors that can be used for vital sign measurement as described herein.

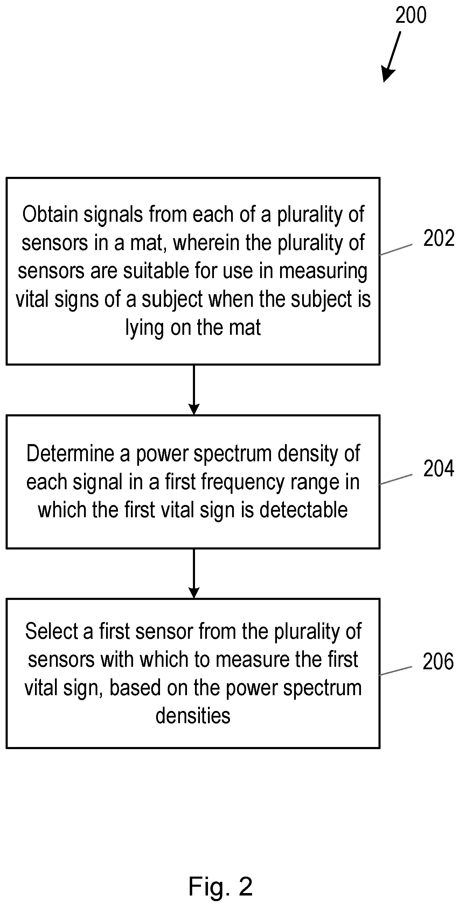

Turning to Fig. 2, there is a computer implemented method 200 for use in measuring a first vital sign of a subject. Embodiments of the method 200 may be performed, for example by an apparatus such as the apparatus 100 described above.

Briefly, in a first step 202, the method 200 comprises: obtaining signals from each of a plurality of sensors in a mat, wherein the plurality of sensors are suitable for use in measuring vital signs of a subject when the subject is lying on the mat. In a second step 204 the method comprises determining a power spectrum density of each signal in a first frequency range in which the first vital sign is detectable and in a third step 206 the method comprises selecting a first sensor from the plurality of sensors with which to measure the first vital sign, based on the power spectrum densities.

In more detail, vital signs include, for example, the heart rate, and/or respiratory rate of the subject. The subject may generally be a patient, e.g. under observation. The subject may be a neonate, infant, child, adult or an animal.

The mat may comprise any of the types of mats described above with respect to the apparatus 100, for example, a sleep mat for a neonate or infant, a mat for epilepsy seizure prediction, or any other mat suitable for use in the methods described herein.

The mat contains a plurality of sensors that are suitable for use in measuring vital signs of a subject, for example, when the subject is lying on the mat. The sensors may comprise, for example, pressure sensors that measure changes in force exerted on the mat. Examples include but are not limited to piezoelectric sensors, such as piezo touch pressure plates, piezo-electric pressure sensory units, accelerometers, fibre optic sensors and proximity sensors.

The sensors may be arranged in the mat in any number of different formations. As an example, they may be arranged in a grid, or matrix like structure.

In some embodiments, the sensors may be of different sensitivities, for example, a first subset of the plurality of sensors may be sensitive to motions in a frequency range appropriate for measuring the heart rate or respiratory rate of an infant, whilst other sensors may be sensitive to motions in a frequency range appropriate for measuring the heart rate or respiratory rate of an adult. Such mixed sensor arrays may enable the mat to be used to monitor adults or children, instead of requiring different mats for subjects of different ages. A combination of lower and higher sensitive embedded pressure sensors also enable both micro and macro movements of the body to be captured.

Frequency ranges appropriate for measuring the heart rate of an adult depend on age, but an appropriate range may be, for example, from about 0.6Hz to about 3Hz. For the respiratory rate, an appropriate range may be from about 0.17Hz to about 0.5 Hz. The skilled person will appreciate however that these ranges are examples only and that other ranges may also be appropriate.

The signals obtained in step 202 may generally be obtained in real-time, or near real time in a continuous manner for the purpose of ongoing monitoring of the subject. For example, the step of obtaining 202 signals may comprise receiving the signals from the mat, e.g. via a wired or wireless communication method.

It will be appreciated that the apparatus 100 and the method 200 may alternatively used for analysis of historical signals, for example, obtained from a database.

In step 204 the method comprises determining a power spectrum density of each signal in a first frequency range in which the first vital sign is detectable.

In some embodiments the step 204 may comprise taking a first Fourier transform (H(t)) of the signal (x(t)) from each of the plurality of sensors (e.g. each of the channels).

$$H(t) = F\big(x(t)\big)$$

The power spectrum may then be computed by integrating the Fourier transform within a passband frequency suitable for measuring the vital sign.

In some embodiments, the passband frequency may be predefined, or pre-set. For example, in an embodiment where the first vital sign is the heart-rate, and the subject is a neonate or infant, then step 204 may comprise determining the power spectrum density in the range $1.5 \, Hz < f < 3.3$ Hz which corresponds to the heartbeat signal of a neonate or infant. In an embodiment where the first vital sign is the respiratory rate, and the subject is a neonate or infant, then step 204 may comprise determining the power spectrum density in the range 0.3 $Hz < f < 1$ $Hz$. It will be appreciated however that the ranges herein are examples only and that different ranges may be selected depending on the subject and vital sign under consideration.

In some embodiments, the method 200 may comprise determining an appropriate range with which to determine the power spectrum density. In other words, in some embodiments, an appropriate frequency range may be determined dynamically, rather than being hard-coded. For example, a frequency spectrum may be determined from a signal from a third sensor of the plurality of sensors in the mat and used to determine the first frequency range in which the first vital sign is detectable, based on a dominant peak in the frequency spectrum.

Dominant peaks are those peaks that carry more energy compared to other peaks in the spectrum. Generally, the frequency spectrum of each sensor in the plurality of sensors will comprise peaks corresponding to the heart-beat and respiration of the subject. The frequency range of the respiratory rate is generally less than the frequency range of the heart rate. Thus, in general, there will be two dominant peaks in the spectrum: one corresponding to the lower frequency range and one corresponding to a relatively higher frequency range. The lower frequency peak may thus be attributed to the respiratory signal whereas the higher frequency peak may be attributed to the heart rate. There may also be other (e.g. noise) peaks due e.g. to factors such as movement of the subject and/or external vibration of the mattress.

The frequency of the dominant peak may be determined by a "third" sensor that may be any one of the sensors in the mat. For example, a signal from an arbitrary sensor may be used to determine the dominant peak and determine an appropriate frequency range for the subject. Thus, it will be appreciated that the third sensor may be different or the same as the first sensor that is subsequently selected in step 206 to monitor the vital sign.

The power spectrum density of the respective signal may be measured in a pre-determined interval about the dominant peak corresponding to the first vital sign. For example, about +/-1.5 Hz of the peak frequency. This is only an example, however, and other frequency ranges may be used, depending on factors such as the age of the subject.

Alternatively, the method may comprise fitting a curve to the dominant peak. A fit may be made using a polynomial curve and a regression method (e.g. such as an nth order polynomial). The power spectrum density of the respective signal may then be measured under the fitted curve.

In other words, frequency spectrum analysis may be used to determine the appropriate frequency range with which to measure the vital sign. In this way, some embodiments of the method 200 are adaptive in that the mat may determine an appropriate frequency range for the individual subject without prior knowledge of the subject's age. This has the advantage of enabling the mat to be used across a wider range of ages of subjects in a fully automated manner.

Once the frequency range or passband is determined then the power spectrum can be determined within said range. For example, in an embodiment where the frequency range 1.5 $Hz < f < 3.3$ $Hz$ is used to determine the heart rate and the frequency range 0.3 $Hz < f < 1$ $Hz$ is used to measure the respiratory rate, the power spectrum in the respective ranges may be computed according to:

$$w_{HR}(t) = 20 * log10(|H(t)|_{1.5\,Hz<f<3.3\,Hz})$$

$$w_{RR}(t) = 20 * log10(|H(t)|_{0.3\,Hz<f<1\,Hz})$$

In step 206 the method then comprises selecting a first sensor from the plurality of sensors with which to measure the first vital sign, based on the power spectrum densities.

As an example, step 206 may comprise selecting the sensor having the highest power spectrum density as the first sensor with which to determine the first vital sign. In this way the sensor with the strongest signal is used to measure the first vital sign.

In one example, a score for each sensor may be determined by computing the median of the energy in the corresponding frequency band. E.g. for the example above, for HR the bandwidth used to compute the median is 1.5 - 3.3 Hz and for RR the bandwidth used to compute the median is 0.3 - 1 Hz.

$$score_{HR} = median\big(w_{HR}(t)\big)$$

$$score_{RR} = median\big(w_{RR}(t)\big)$$

Such scores may be used to select the first sensor from the plurality of sensors with which to measure the first vital sign. For example, the sensor having the highest score may be selected.

It will be appreciated however that this is merely an example, and that the n sensors having the highest scores may be used and e.g. averaged to obtain the vital sign measurements. In this way, the sensors receiving the strongest signals may be selected and combined in order to improve the vital sign measurement.

It will be further appreciated that more than one vital sign may be measured, for example steps 204 and 206 may be repeated in order to determine a second sensor with which to determine a second vital sign. In other words, the method 200 may further comprise: determining a power spectrum density of each signal in a second frequency range in which a second vital sign is detectable, and selecting a second sensor with which to measure the second vital sign based on the power spectrum densities in the second frequency range. The second frequency range may be determined using any of the methods described above with respect to the first frequency range.

Once the optimal sensor(s) have been determined with which to determine the first (and subsequent) vital signs, then the signals from said sensors may be processed in order to determine the vital sign signal. The skilled person will be familiar with methods of determining a vital sign such a heart rate or respiratory rate from the sensors described herein (e.g. pressure sensors). An example method may be found in the paper by Lee et al. (2015) entitled, "Ballistocardiogram of baby during sleep" 2015 37th Annual International Conference of the IEEE Engineering in Medicine and Biology Society (EMBC), Milan, Italy, 2015, pp. 7167-7170.

Turning now to other embodiments, various preprocessing steps may be performed in order to further improve the vital sign measurement. For example, in some embodiments, the method 200 may be performed during a period of inactivity of the subject.

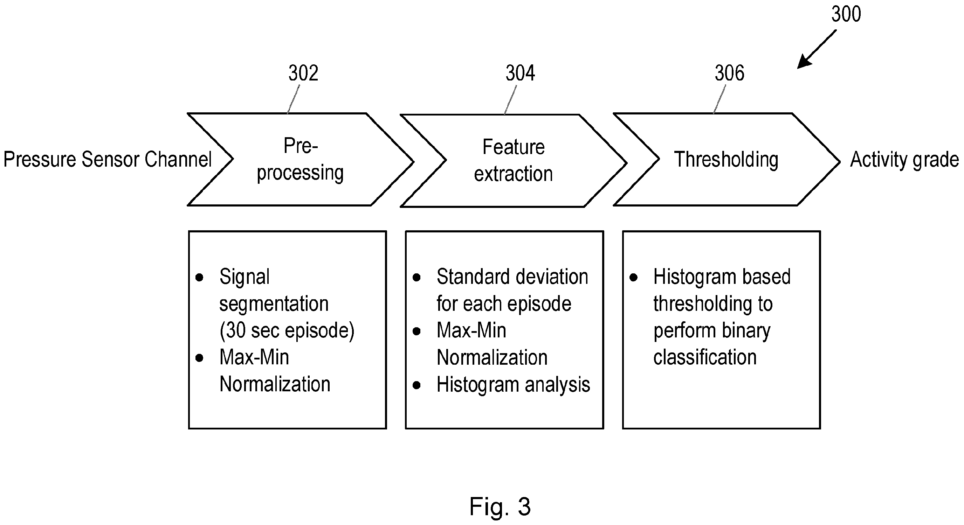

This is illustrated in Fig. 3 which shows a method of preprocessing the signals obtained in step 202 of the method 200 according to some embodiments herein. The purpose of the method of Fig 3 is activity classification with the primary objective of this step being to identify a reliable time interval within which the signal can be used for effective vital sign monitoring. The method 300 may thus be performed between steps 202 and 204 in order to determine an appropriate time window in which to measure the first vital sign (e.g. perform step 206).

In step 302 the method 300 comprises segmenting (e.g. splitting) the signal from the first sensor into a plurality of time windows. For example, the signal may be split into 30 second windows, or episodes. The skilled person will appreciate that this is merely an example, and that other length windows might also be used, depending on factors such as the activity level of the subject and/or the duration of monitoring.

Normalization may then be performed, so that the maximum amplitude in each window is scaled to a constant value (e.g. 1.0). This enables more straightforward comparison of the signal between windows.

In step 304 the method then comprises determining a measure of change in amplitude in the signal between each window. For example, in some embodiments the standard deviation of the signal in each window is determined. The standard deviation measures are then compared between windows. This is merely an example however and any measure of the magnitude or change or intensity between windows could be used. Examples of measures of change include, but are not limited to, power, energy, and entropy as well as the aforementioned standard deviation. In this way, the magnitude/change or intensity between windows is determined.

In step 306 the method then comprises determining a period of inactivity in which to measure the first vital sign, based on the determined measures of change.

This may comprise performing histogram analysis, for example, on peaks in the measure of change in amplitude (standard deviation, entropy etc) over time. The processor may be further configured to determine an activity score for each time window, indicating the relative activity level of the subject in the time window, compared to other time windows. For example, thresholds may be set in order to classify different peaks and thus different time windows as "active" or "inactive". The processor may then be further configured to determine the vital sign during the period of inactivity. In this way, a period of inactivity may be found in which to perform the method 200 described above and obtain vital sign measurements that are less influenced by noise caused by movement of the patient.

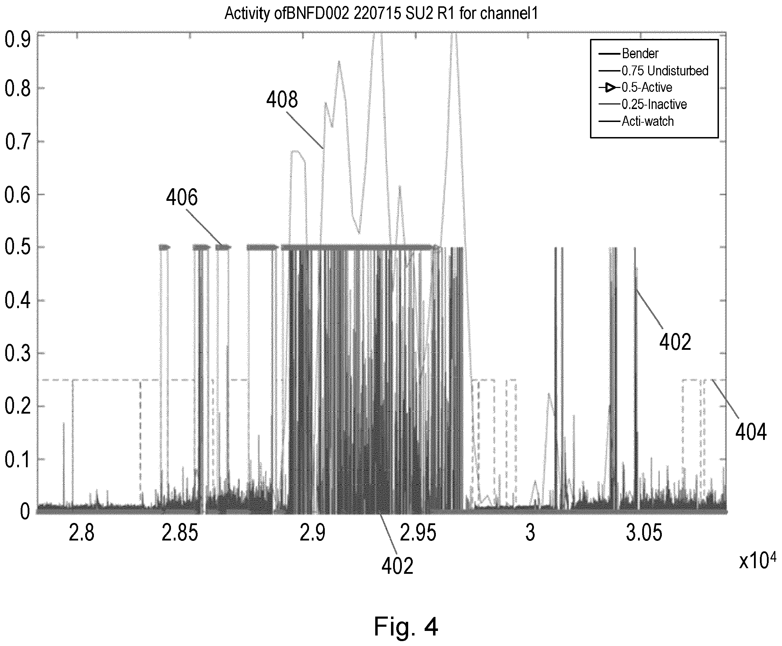

This is illustrated in Fig. 4 which shows the output of the method 300. In Fig. 4 signal data is illustrated by the dark lines 402. windows of inactivity are illustrated by the dashed lines 404, whilst the thick dark lines 406 illustrate periods of activity. Activity as measured using an activity watch is shown in the light grey lines 408. The over-lap between the periods of activity as determined by the activity watch (peaks in the line 408) and the method 300 (indicated by the lines 406) show good over-lap, validating the method.

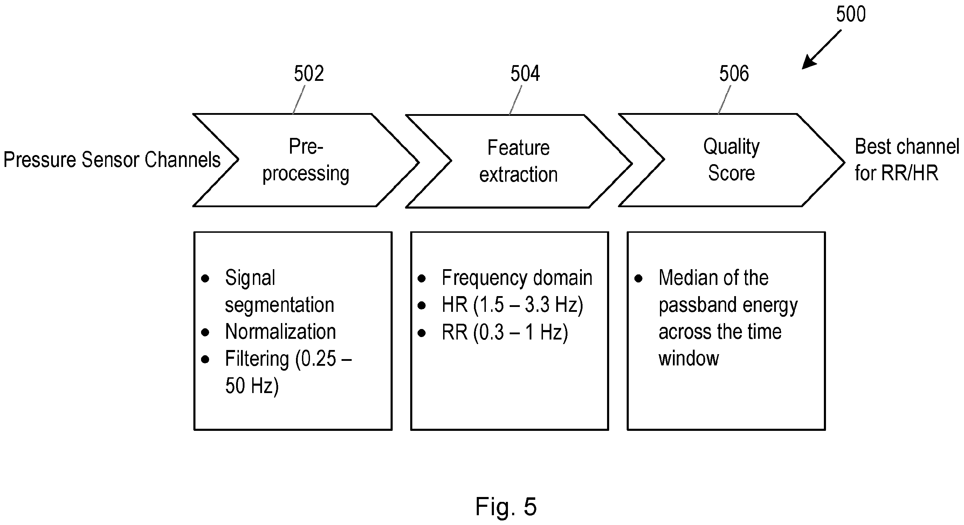

Turning now to Fig. 5 which shows an example implementation of the method 200 according to an embodiment. The method 500 is suitable for measuring a first vital sign corresponding to the heart rate and a second vital sign corresponding to the respiratory rate of a neonate lying on the mat. The method 500 may be performed, e.g., on portions of the signal where the subject is determined to be inactive, according to the method 300 described above.

In this embodiment, in step 502 the signal from each of the plurality of sensors is segmented into windows and normalized (e.g. within each window).

The signal from each window is then filtered in order to remove frequencies outside of a predefined filtering range. In this example, frequencies outside the range 0.25-50Hz are filtered, but it will be appreciated that this is merely an example and that other ranges could be used instead.

In step 504 a Fourier transform is performed on the signals from each of the plurality of sensors as described above with respect to step 204. In step 504, the power spectrum density of each signal in a frequency band encompassing movements due to the heart beat is determined 204. For neonates and infants an appropriate range is 1.5 Hz<f<3.3 Hz for the heart beat.

The median of the passband energy across each time window for each sensor is then determined as described above and used as a score with which to rank each sensor. The best channel/sensor with which to measure the heart rate is chosen as that having the highest median passband energy.

For the respiratory rate, in step 504, the power spectrum density of each signal in a frequency band encompassing movements due to respiration is also determined 204. For neonates and infants an appropriate range is 0.3 Hz<f<1 Hz.

The median of the passband energy across each time window for each sensor is then determined as described above and used as a score with which to rank each sensor. The best channel/sensor with which to measure the respiratory rate is chosen as that having the highest median passband energy.

It is noted that in some scenarios, different sensors may be chosen to monitor each vital sign. The skilled person will also appreciate that the method 500 can be modified to detect the heart rate and respiratory rate of subjects of different ages by changing the frequency ranges described above.

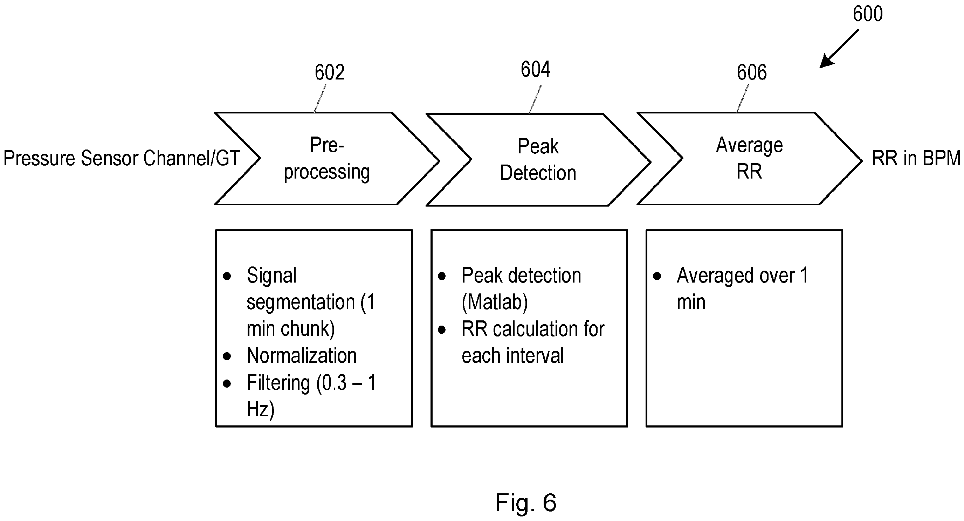

Fig. 6 shows another example implementation of the method 200 according to an embodiment. The method 600 is suitable for measuring a first vital sign corresponding to the heart rate and a second vital sign corresponding to the respiratory rate of any subject (e.g. a subject of any age) lying on the mat. In some embodiments, the method 600 may be performed, e.g., on portions of the signal where the subject is determined to be inactive, as determined according to the method 300 described above.

In this embodiment, in step 602 the signals from each of the plurality of sensors are segmented, e.g. into windows. In this example, the windows are 1 minute long, although it will be appreciated that other length windows could equally be used. Normalisation is then performed and filtering to remove signals outside the frequency range 0.3-1Hz.

A fourier transform is performed on each signal to determine a frequency spectrum. A frequency range with which to measure the vital sign is then determined, based on the dominant peaks in the frequency spectrum. To this end, peak detection is performed to determine the dominant peaks. As described above with respect to step 206 of the method 200, based on the dominant peaks, a predefined interval may be defined either side of the dominant peaks within which to measure the power spectrum density. Alternatively, a curve may be fitted to each peak and the power spectrum density may thus be determined by integrating under the fitted curve. In this way the method may be automatically adapted to measure the vital signs of a wide range of different subjects, e.g. infants, neonates, adults or animals in the manner of a Biologically triggered vital sign estimation.

Thus according to embodiments herein, in order to measure a specific vital sign, a sensor/sensors may be chosen based on the power spectrum density in the specific frequency range suitable for the particular vital sign (RR and HR). This improves accuracy of the vital sign measurement.

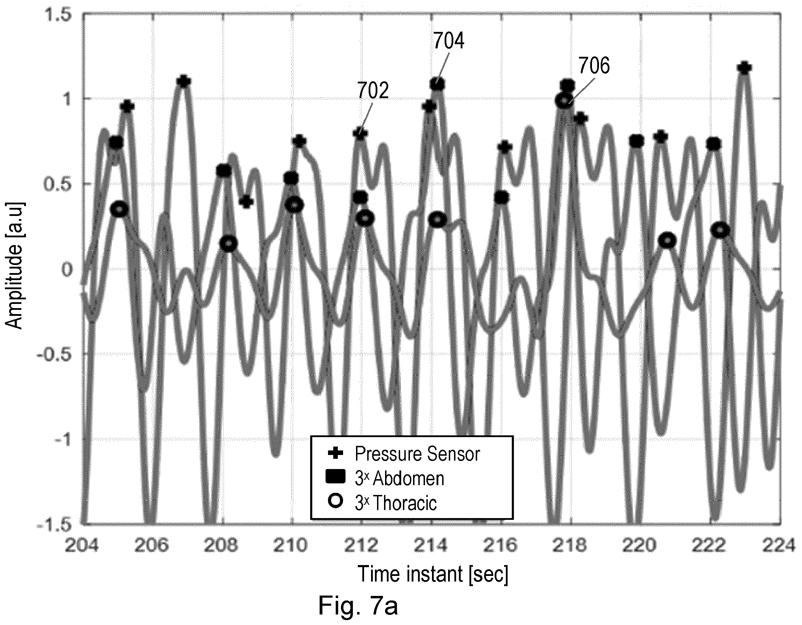

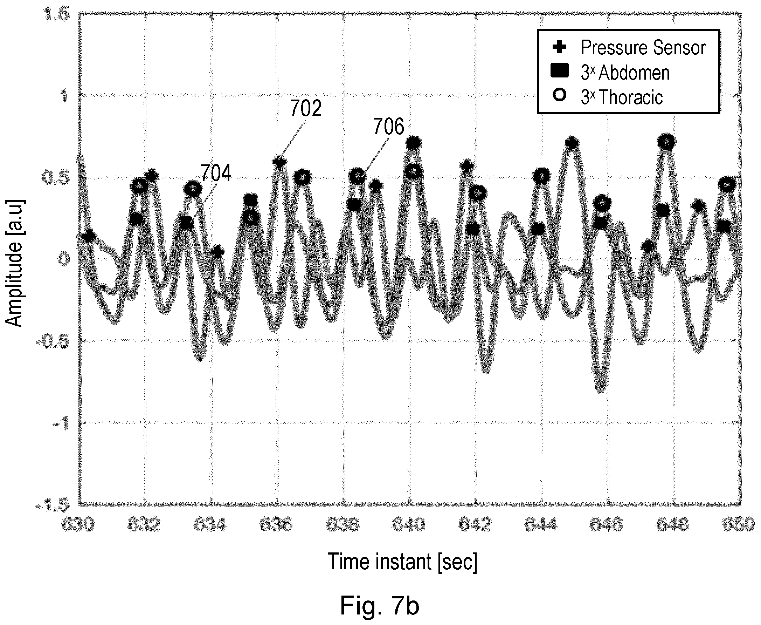

Fig. 7a and 7b illustrate respiratory signals of two patients. Respiratory signals extracted from sensor data using the method 200 are shown by the cross symbols, alongside ground truth data obtained using an abdominal belt (filled squares) and a thoracic belt (open squares). The good alignment between the results of the method 200 and the ground truth data illustrate the efficacy of the embodiments herein.

Turning now to another embodiment, as noted above in some embodiments, the mat described herein may be for use in seizure prediction and/or monitoring of subjects with epilepsy. Variability of HR during seizures is a current area of research, see for example, the paper by Ferri et al., entitled "Heart rate variability during sleep in children with partial epilepsy", Journal of Sleep Research, 11(2), pp. 153-60, 2002; the paper by Tomson et al., entitled "Heart rate variability in patients with epilepsy, Epilepsy Research", 30(1), pp.77-83, 1998; see also the paper by Pradhan et al., entitled "Quantitative analysis of heart rate variability in patients with absence epilepsy", Neurol India., 59(1), pp.25-9, 2011. One of the clinical protocols described in these papers is the use of video Electroencephalography, EEG, observed over prolonged time span (as an alternative to positron emission tomography, PET, or Functional magnetic resonance imaging, fMRI), where a doctor observes the patients seizure event patters, lowering the medicine dosage during this period.

In such respect, the methods described herein may be used to determine vital sign measurements for use in the prediction of seizures. A sleep-mat may be a more convenient and less intrusive manner in which to monitor patients for long periods of time in order to identifying and/or predict seizure events.

As such, in some embodiments, the method further comprises processing the first vital sign to detect variability of the first vital sign for use in seizure prediction. The first vital sign may be processed in the manner described in any of the papers above, for example.

In embodiments wherein the method 200 is performed by a system for determining a vital sign of a subject, as described above. Said system may further be configured to produce an alarm/alert or similar upon detection of a seizure. Alternatively or additionally, said system may be configured to propose a change in dosage of a drug in response to detection of a seizure.

Turning now to other embodiments, there is also provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. An apparatus for measuring a first vital sign of a subject, the apparatus comprising:

   a memory comprising instruction data representing a set of instructions; and
   a processor configured to communicate with the memory and to execute the set of instructions, wherein the set of instructions, when executed by the processor, cause the processor to:

   obtain signals from each of a plurality of sensors in a mat, wherein the plurality of sensors are suitable for use in measuring vital signs of a subject when the subject is lying on the mat;
   determine a power spectrum density of each signal in a first frequency range in which the first vital sign is detectable; and
   select a first sensor from the plurality of sensors with which to measure the first vital sign, based on the power spectrum densities.

2. An apparatus according to claim 1 wherein the processor is further caused to:
   select the sensor having the highest power spectrum density as the first sensor with which to determine the first vital sign.

3. An apparatus as in any one of the preceding claims wherein the processor is further caused to:

   determine a power spectrum density of each signal in a second frequency range in which a second vital sign is detectable; and
   select a second sensor with which to measure the second vital sign based on the power spectrum densities in the second frequency range.

4. An apparatus as in claim 1, 2 or 3 wherein the processor is further caused to:

   determine a frequency spectrum of a signal from a third sensor of the plurality of sensors in the mat; and
   determine the first frequency range in which the first vital sign is detectable based on a dominant peak in the frequency spectrum.

5. An apparatus as in claim 4 wherein the power spectrum density of the respective signal is measured in a pre-determined interval about the dominant peak.

6. An apparatus as in claim 4 wherein the processor is further caused to fit a curve to the dominant peak and wherein the power spectrum density of the respective signal is measured under the fitted curve.

7. An apparatus as in any one of the preceding claims wherein the processor is further caused to:

> segment the signal from the first sensor into a plurality of time windows;
> determine a measure of change in amplitude in the signal between each window; and
> determine a period of inactivity in which to measure the first vital sign, based on the determined measures of change.

8. An apparatus as in claim 7 wherein the processor is further caused to determine an activity score for each time window, indicating the relative activity level of the subject in the time window, compared to other time windows.

9. An apparatus as in claim 7 or 8 wherein the processor is further configured to determine the vital sign during the period of inactivity.

10. An apparatus as in any one of the preceding claims wherein the apparatus is further configured to process the first vital sign to detect variability of the first vital sign for use in seizure prediction.

11. A system for determining a vital sign of a subject, the system comprising:

> a mat comprising a plurality of sensors suitable for use in measuring vital signs of a subject when the subject is lying on the mat; and
> an apparatus as in any one of the preceding claims.

12. A system as in claim 11 wherein the mat is a sleep mat for a baby or a neonate.

13. A system as in claim 11 or 12 wherein the system is for use in seizure prediction of a subject.

14. A computer implemented method for measuring a first vital sign of a subject, the method comprising:

> obtaining signals from each of a plurality of sensors in a mat, wherein the plurality of sensors are suitable for use in measuring vital signs of a subject when the subject is lying on the mat;
> determining a power spectrum density of each signal in a first frequency range in which the first vital sign is detectable; and
> selecting a first sensor from the plurality of sensors with which to measure the first vital sign, based on the power spectrum densities.

15. A computer program product comprising a computer readable medium, the computer readable medium having computer readable code embodied therein, the computer readable code being configured such that, on execution by a suitable computer or processor, the computer or processor is caused to perform the method as claimed in claim 14.

100

102

Processor

104

Memory

106

Instructions

Fig. 1

200

```
┌─────────────────────────────────────┐
│ Obtain signals from each of a        │
│ plurality of sensors in a mat,       │        202
│ wherein the plurality of sensors     │
│ are suitable for use in measuring    │
│ vital signs of a subject when the    │
│ subject is lying on the mat          │
└─────────────────────────────────────┘
                    │
                    ▼
┌─────────────────────────────────────┐
│ Determine a power spectrum density   │
│ of each signal in a first frequency  │        204
│ range in which the first vital sign  │
│ is detectable                        │
└─────────────────────────────────────┘
                    │
                    ▼
┌─────────────────────────────────────┐
│ Select a first sensor from the       │
│ plurality of sensors with which to   │        206
│ measure the first vital sign, based  │
│ on the power spectrum densities      │
└─────────────────────────────────────┘
```

Fig. 2

300

302

304

306

Pressure Sensor Channel ⟩ Pre-
processing ⟩⟩ Feature
extraction ⟩⟩ Thresholding ⟩ Activity grade

- Signal
segmentation
(30 sec episode)
- Max-Min
Normalization

- Standard deviation
for each episode
- Max-Min
Normalization
- Histogram analysis

- Histogram based
thresholding to
perform binary
classification

Fig. 3

Activity ofBNFD002 220715 SU2 R1 for channel1

Bender
0.75 Undisturbed
0.5-Active
0.25-Inactive
Acti-watch

404

402

402

408

406

x10⁴

Fig. 4

500

502 504 506

Pressure Sensor Channels → Pre-processing → Feature extraction → Quality Score → Best channel for RR/HR

| Pre-processing | Feature extraction | Quality Score |
|---|---|---|
| • Signal segmentation<br>• Normalization<br>• Filtering (0.25 – 50 Hz) | • Frequency domain<br>• HR (1.5 – 3.3 Hz)<br>• RR (0.3 – 1 Hz) | • Median of the passband energy across the time window |

Fig. 5

EP 4 052 644 A1

Pressure Sensor Channel/GT → **Pre-processing** (602) → **Peak Detection** (604) → **Average RR** (606) → RR in BPM

600

**Pre-processing (602)**

- Signal segmentation (1 min chunk)
- Normalization
- Filtering (0.3 – 1 Hz)

**Peak Detection (604)**

- Peak detection (Matlab)
- RR calculation for each interval

**Average RR (606)**

- Averaged over 1 min

Fig. 6

Fig. 7a

Fig. 7b

Europäisches
Patentamt

European
Patent Office

Office européen
des brevets

# EUROPEAN SEARCH REPORT

Application Number

EP 21 16 0464

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/193357 A1 (KONINKLIJKE PHILIPS NV [NL]) 1 October 2020 (2020-10-01) | 1-3,6, 10-15 | INV. A61B5/024 A61B5/113 A61B5/00 A61B5/11 |
| Y | * paragraphs [0040], [0041], [0077] - [0086] * | 4,5,7-9 | |
| Y | KR 102 139 047 B1 (PEOPLE MULTI CO LTD [KR]) 30 July 2020 (2020-07-30) * paragraphs [0067], [0068] * | 4,5 | |
| A | US 5 479 932 A (HIGGINS JOSEPH [US] ET AL) 2 January 1996 (1996-01-02) * column 6, lines 34-67 * | 4,5 | |
| Y | US 2012/184825 A1 (BEN DAVID MEIR [IL]) 19 July 2012 (2012-07-19) * paragraphs [0060] - [0063] * | 7-9 | |
| A | EP 3 769 674 A1 (TNO [NL]) 27 January 2021 (2021-01-27) * paragraph [0012] * | 7-9 | |

TECHNICAL FIELDS
SEARCHED (IPC)

A61B

The present search report has been drawn up for all claims

| Place of search | Date of completion of the search | Examiner |
|---|---|---|
| Berlin | 9 August 2021 | Trachterna, Morten |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 21 16 0464

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2021

| Patent document cited in search report | | Publication date | Patent family member(s) | | Publication date |
|---|---|---|---|---|---|
| WO 2020193357 | A1 | 01-10-2020 | NONE | | |
| KR 102139047 | B1 | 30-07-2020 | KR 102139047 B1 | | 30-07-2020 |
| | | | KR 20210077576 A | | 25-06-2021 |
| US 5479932 | A | 02-01-1996 | NONE | | |
| US 2012184825 | A1 | 19-07-2012 | NONE | | |
| EP 3769674 | A1 | 27-01-2021 | EP 3769674 A1 | | 27-01-2021 |
| | | | WO 2021015617 A1 | | 28-01-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEE et al.** Ballistocardiogram of baby during sleep. *2015 37th Annual International Conference of the IEEE Engineering in Medicine and Biology Society (EMBC),* 2015, 7167-7170
- **FERRI et al.** Heart rate variability during sleep in children with partial epilepsy. *Journal of Sleep Research,* 2002, vol. 11 (2), 153-60

- **TOMSON et al.** *Heart rate variability in patients with epilepsy, Epilepsy Research,* 1998, vol. 30 (1), 77-83
- **PRADHAN et al.** Quantitative analysis of heart rate variability in patients with absence epilepsy. *Neurol India.,* 2011, vol. 59 (1), 25-9